(19) Europäisches Patentamt
European Patent Office
Office européen des brevets

(11) Numéro de publication: **0 433 178 B1**

(12) **FASCICULE DE BREVET EUROPEEN**

(45) Date de publication de fascicule du brevet: **28.12.94**

(51) Int. Cl.$^{5}$: **C07B 57/00**, C07D 323/00, B01J 19/00

(21) Numéro de dépôt: **90403578.9**

(22) Date de dépôt: **13.12.90**

(54) **Procédé de séparation des isomères d'un éther-couronne, permettant de récupérer en particulier l'isomère cis-syn-cis pur du dicyclohexyl 18-couronne 6-DCH 18C6.**

(30) Priorité: **15.12.89 FR 8916637**

(43) Date de publication de la demande:
**19.06.91 Bulletin 91/25**

(45) Mention de la délivrance du brevet:
**28.12.94 Bulletin 94/52**

(84) Etats contractants désignés:
**BE DE FR GB SE**

(56) Documents cités:

**CHEMICAL ABSTRACTS vol. 110, no. 7, 13 février 1989, page 654, abrégé no. 67297k, Columbus, Ohio, US; P.J. ZHENG et al.: "Structural study of extraction complexes of uranium with dicyclohexyl-18-crown-6 isomer A" & Huaxue Xuebao 1988, vol. 46, no. 9, pages 837-843**

(73) Titulaire: **COGEMA COMPAGNIE GENERALE DES MATIERES NUCLEAIRES**
**2, rue Paul Dautier**
**B.P. 4**
**F-78141 Velizy-Villacoublay (FR)**

(72) Inventeur: **Lemaire, Marc**
**23 rue Château Gaillard**
**F-69100 Villeurbanne (FR)**
Inventeur: **Guy, Alain**
**1 rue Urgons**
**F-77135 Pontcarre (FR)**
Inventeur: **Foos, Jacques**
**33 rue Louis Scocard**
**F-91400 Orsay (FR)**
Inventeur: **Guyon, Vincent**
**235 rue Saint Martin**
**F-75005 Paris (FR)**
Inventeur: **Chomel, Rodolphe**
**27 Bis, Avenue de Champlin**
**F-84100 Orange (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**CHEMICAL ABSTRACTS vol. 102, no. 1, 7 janvier 1985, page 599, abrégé no. 6565j, Columbus, Ohio, US; N.A. TSARENKO et al.: "Separation of cis-syn-cis and cis-anti-cis isomers of dicyclohexyl-18-crown-6" & Otkrytiya, Izobret.; Prom. Obraztsy, Tovarnye Znaki 1984, no. 27,**

**CHEMICAL ABSTRACTS vol. 108, no. 3, 18 janvier 1988, page 607, abrégé no. 21936y, Columbus, Ohio, US; V.V. YAKSHIN et al.: "Preparation and separation of trans-syn-trans and trans-anti-trans-isomers of dicyclohexano-18-crown-6" & Otkrytiya, Izobret. 1986, no. 42, pages**

**INORGANIC CHEMISTRY vol. 14, no. 12, décembre 1975, pages 3132,3133; R.M. IZATT et al.: "Facile Separation of the Cis Isomers of Dicyclohexal-18-crown-6"**

(74) Mandataire: **Mongrédien, André et al**
**c/o BREVATOME**
**25, rue de Ponthieu**
**F-75008 Paris (FR)**

## Description

La présente invention a pour objet un procédé de séparation des isomères des éthers-couronnes, qui permet d'isoler en particulier l'isomère cis-syn-cis du DCH 18C6.

De façon plus précise, elle concerne la séparation des isomères des éthers-couronnes de formule:

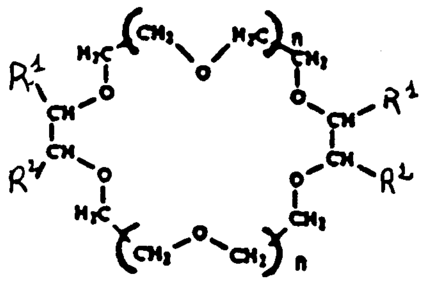

(I)

dans laquelle n = 0 ou est un nombre entier allant de 1 à 3, $R^1$ et $R^2$ qui sont identiques ou différents sont des radicaux alkyle ou alcoxyalkyle, ou $R^1$ et $R^2$ forment ensemble un radical cycloalkyle.

Parmi ces éthers-couronnes, le dicyclohexyl 18-Crown-6 (DCH18C6) qui répond à la formule:

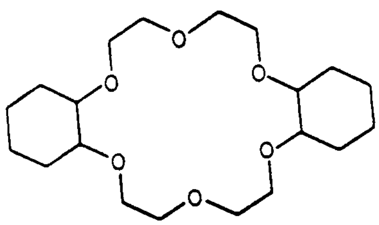

(II)

est un produit disponible dans le commerce sous la forme d'un mélange de plusieurs isomères.

En effet, ce produit possède 5 diastéréo isomères qui ont les structures suivantes:

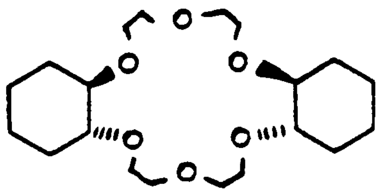

trans-syn-trans

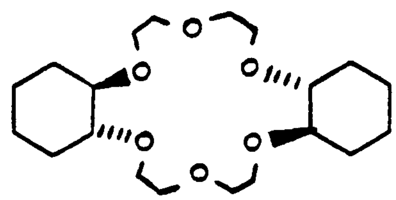

trans-anti-trans

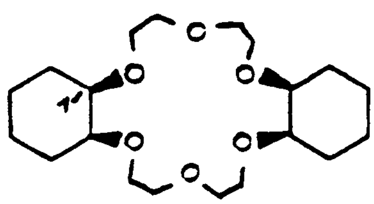

cis-syn-cis A

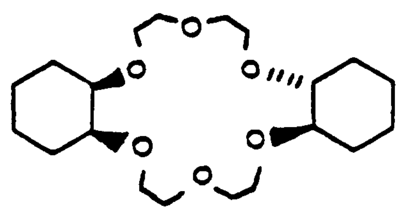

cis-trans

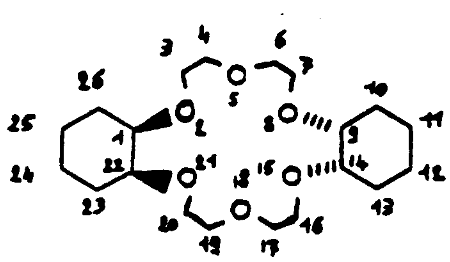

forme cristalline $B_1$: 69-70°C

forme cristalline $B_2$: 83-84°C

cis-anti-cis B

Lorsque le DCH18C6 est obtenu par hydrogénation catalytique du dibenzo-18-Crown-6, comme c'est le cas des produits commerciaux, il contient principalement les isomères cis-syn-cis (isomère A) et cis-anti-cis (isomère B).

Pour certaines applications, il peut être intéressant d'utiliser l'un des isomères purs plutôt que le mélange d'isomères. Ainsi le document Chemical Abstracts : 110, n° 7, 1989, réf. 67297 k décrit l'utilisation de l'isomère A (cis-syn-cis) du DCH18C6 pour extraire $UO_2Cl_2$ et $UCl_4$ d'une solution chlorhydrique.

Cependant, les procédés connus jusqu'à présent pour séparer les différents isomères du DCH18C6 sont difficiles à mettre en oeuvre à une échelle industrielle, avec des rendements satisfaisants.

Parmi ces procédés, on connaît un procédé de séparation des isomères du DCH18C6 par chromatographie sur une colonne d'alumine, utilisant un mélange hexane-éther dont le gradient de concentration en éther augmente au cours du temps pour éluer l'isomère A, et du méthanol pour recueillir l'isomère B. Ce procédé décrit par R.M. Izatt et col dans J. Amer. Chem. Soc., 93, 1619 (1971), présente l'inconvénient d'être long et coûteux à mettre en oeuvre, et de ne conduire qu'à des rendements de 20 à 30% pour chacun des isomères.

R.N. Izatt et col ont décrit dans Inorganic Chemistry, 14, 3132 (1975), un autre procédé de séparation des isomères du DCH18C6 par une méthode basée sur la précipitation sélective du complexe DCH18C6/perchlorate de plomb de l'isomère B (cis-an-ti-cis).

Après séparation du précipité comprenant l'isomère B par filtration et traitement du filtrat avec $H_2S$ pour éliminer le plomb, on récupère l'isomère A (cis-syn-cis) restant en solution par addition d'acide perchlorique pour précipiter l'isomère A. Bien que cette méthode conduise à des rendements plus élevés, elle ne peut être utilisée facilement à l'échelle industrielle, car elle nécessite l'emploi de perchlorates de métaux lourds connus pour être explosifs et d'hydrogène sulfuré connu pour être toxique.

Un autre procédé de séparation des isomères du DCH18C6 décrit par C.J. Pedersen dans Organic Syntheses, 52, 66, utilise la cristallisation du complexe DCH18C6/acetate de potassium dans un mélange $CH_2Cl_2$/ether de pétrole.

Ce procédé présente l'inconvénient de convenir seulement à la séparation de l'isomère B (cis-anti-cis) et de ne conduire qu'à un rendement de 12%.

Ainsi, les procédés connus de séparation des isomères du DCH18C6 sont, soit peu performants, soit coûteux et longs, soit impossibles à mettre en oeuvre à l'échelle industrielle.

La présente invention a précisément pour objet un procédé de séparation des isomères d'un éther-couronne tel que le DCH18C6 qui pallie ces inconvénients.

Selon l'invention, le procédé de séparation des isomères d'un ether-couronne de formule:

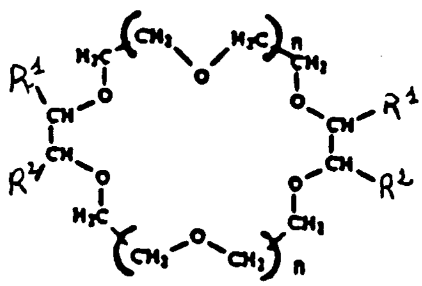

(I)

dans laquelle n = 0 ou est un nombre entier allant de 1 à 3, $R^1$ et $R^2$ qui sont identiques ou différents, représentent un radical alkyle, un radical alcoxyalkyle ou un radical poly(alcoxyalkyle) ou $R^1$ et $R^2$ forment ensemble un radical cycloalkyle, caractérisé en ce qu'il comprend les étapes successives suivantes :

a) dissoudre dans un solvant organique un mélange des isomères dudit éther-couronne comprenant l'isomère cis-anti-cis et l'isomère cis-syn-cis,

b) ajouter du nitrate d'uranyle à la solution obtenue dans l'étape a) en quantité telle que l'on précipite pratiquement tous les isomères de l'éther-couronne sauf l'isomère cis-syn-cis, sous la forme de complexes avec le nitrate d'uranyle,

c) séparer le précipité ainsi formé, et

d) récupérer l'isomère cis-syn-cis pur restant dans la solution.

Dans la formule (I) donnée ci-dessus $R^1$ et $R^2$ peuvent être identiques ou différents et représentent des radicaux alkyle, alcoxyalkyle ou poly(alcoxyalkyle).

Les radicaux alkyle et alcoxyalkyle peuvent être linéaires ou ramifiés et comporter de 1 à 10 atomes de carbone. A titre d'exemples de tels radicaux, on peut citer les radicaux méthyle, éthyle, propyle, isopropyle, butyle, tert-butyle, isobutyle, méthoxyméthyle, éthoxyméthyle, méthoxyéthyle, méthoxybutyle.

Les radicaux poly(alcoxyalkyle) sont des radicaux alkyle comportant plusieurs fonctions éther-oxyde. Ils peuvent répondre à la formule :

$-R^3-X-R^4$

dans laquelle $R^3$ est un radical alcoylène bivalent, par exemple méthylène, X est un radical polyoxyalkylène dérivé de polyéthylèneglycols, par exemple le radical de formule :

$-[(CH_2)_n-O]_r - [(CH_2)_m - O]_s - (CH_2)_p -$

dans laquelle n, m, p, r et s sont des nombre entiers allant de 1 à 4, et $R^4$ est un radical alkyle.

Lorsque $R^1$ et $R^2$ forment ensemble un radical cycloalkyle, il a de préférence 3 à 6 atomes de carbone.

Le mode de séparation utilisé dans le procédé de l'invention repose sur la différence de solubilité dans le solvant organique utilisé, de l'isomère cis-syn-cis et du complexe formé entre l'isomère cis-anti-cis et le nitrate d'uranyle.

Il est basé également sur l'emploi d'une quantité appropriée de nitrate d'uranyle pour s'assurer que tous les isomères de l'éther-couronne à l'exception de l'isomère cis-syn-cis sont précipités sous forme de complexes alors que l'isomère cis-syn-cis reste en solution.

La quantité de nitrate d'uranyle à utiliser dépend en particulier de la nature de l'éther-couronne, des conditions de précipitation et de la teneur en isomère B du mélange. Dans le cas du DCH18C6, on obtient de bons résultats lorsque l'on utilise 3,5 mol de nitrate d'uranyle par mol d'isomère B.

La quantité optimale peut être déterminée facilement par des essais de routine, comme on le verra ci-après.

Dans ce procédé, la quantité de nitrate d'uranyle à utiliser dépend de la teneur en isomère B (cis-anti-cis) du mélange de départ.

Aussi, il est nécessaire de déterminer préalablement la teneur en isomère B. Celle-ci peut être évaluée par exemple par analyse du mélange par résonance magnétique nucléaire.

Généralement, les mélanges commerciaux d'isomères d'éthers-couronnes tels que le DCH18C6 contiennent de 46 à 65% d'isomère cis-syn-cis et de 37 à 40% d'isomère cis-anti-cis.

Selon un mode préféré de mise en oeuvre du procédé de l'invention, on réalise tout d'abord une étape préalable consistant à séparer une partie de l'isomère B (cis-anti-cis) présent dans le mélange d'isomères de départ pour traiter un mélange enrichi en l'isomère recherché, soit l'isomère A, et utiliser de ce fait une quantité moindre de nitrate d'uranyle.

Cette étape préalable peut être réalisée par dissolution du mélange dans un solvant organique, cristallisation de l'isomère B présent dans cette solution et séparation de l'isomère B ainsi cristallisé.

En effet, on a remarqué que lorsqu'on dissout un mélange d'isomères d'éthers-couronnes dans un solvant approprié, et que l'on effectue ensuite une recristallisation à la température ambiante, le produit cristallisé est constitué d'isomère B pur, sous la forme cristalline $B_1$ dans le cas du DCH18C6 ou est enrichi en isomère B. Par conséquent, la solution s'est enrichie en isomère A par rapport au mélange initial.

Les solvants organiques qui permettent d'obtenir un tel enrichissement peuvent être les hydrocarbures saturés aliphatiques ou les hydrocarbures aromatiques.

A titre d'exemple d'hydrocarbures saturés aliphatiques, on peut citer le pentane, l'heptane et l'isooctane. Dans le cas du DCH18C6, l'heptane et le pentane conduisent à la cristallisation d'isomère B pur alors que l'isooctane conduit à un produit cristallisé contenant 14,7% d'isomère A; de préférence, on utilise l'heptane qui est plus efficace que le pentane pour enrichir la solution en isomère A.

Les solvants organiques utilisés pour l'étape de précipitation des complexes avec le nitrate d'uranyle sont du même type puisque le principe de séparation de ces complexes est également basé sur une différence de solubilité des complexes dans le solvant.

Aussi, dans le cas où l'éther-couronne est le DCH18C6, on utilise de préférence également l'heptane pour l'étape b) de précipitation.

Selon le procédé de l'invention, on peut récupérer l'isomère B) (cis-anti-cis) à partir du précipité séparé dans l'étape c).

Dans ce but, on peut décomposer le complexe par traitement à l'eau et au chloroforme pour libérer l'isomère B pur et récupérer le nitrate d'uranyle qui peut ainsi être réutilisé.

L'isomère A qui reste en solution peut être récupéré à l'état pur par évaporation du solvant.

Ainsi, le procédé de l'invention est facile à mettre en oeuvre. Par ailleurs, les réactifs utilisés ne posent aucun problème de sécurité ou d'approvisionnement. En effet, le réactif principal est du nitrate d'uranyle appauvri qui est un sous-produit des usines d'enrichissement isotopique de l'uranium.

D'autres caractéristiques et avantages de l'invention apparaîtront mieux à la lecture des exemples suivants donnés bien entendu à titre illustratif et non limitatif en référence au dessin annexé sur lequel :

- la figure 1 est un diagramme illustrant un mode de mise en oeuvre du procédé de l'invention, et
- la figure 2 est un spectre de RMN $^{13}C$ d'un mélange commercial d'isomères du DCH18C6.

Sur la figure 1, on a représenté de façon schématique un mode de mise en oeuvre du procédé de l'invention comportant l'étape préliminaire de recristallisation de l'isomère cis-anti-cis.

Comme il est représenté sur la figure 1, on réalise tout d'abord l'étape de recristallisation de l'isomère B (cis-anti-cis).

Dans ce but, on dissout le mélange commercial d'isomères du DCH18C6 dans un solvant approprié tel que l'heptane, puis on recristallise l'isomère B à partir de cette solution à la température ambiante.

Après cette recristallisation, on sépare les cristaux de l'isomère B par filtration de la solution, puis on évapore la solution pour recueillir un mélange d'isomères du DCH18C6 enrichi en isomère A (cis-syn-cis).

Pour réaliser l'étape suivante de précipitation par le nitrate d'uranyle, on analyse tout d'abord le mélange par résonance magnétique nucléaire du $^{13}C$ pour déterminer sa teneur en isomère B.

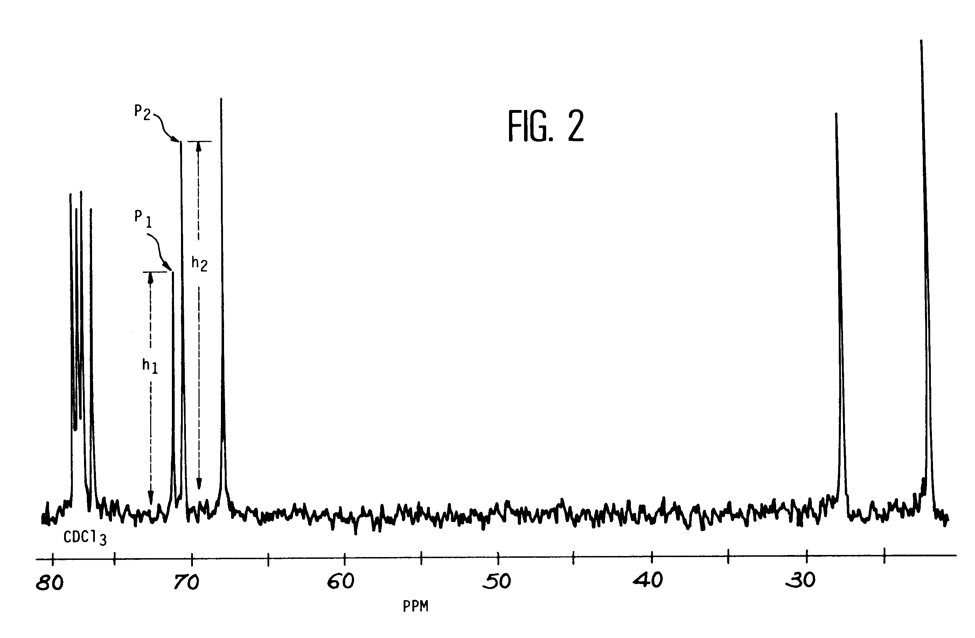

On obtient ainsi un spectre tel que celui représenté sur la figure 2. Dans ce spectre, le pic $P_1$ correspond à l'isomère A, alors que le pic $P_2$ correspond à l'isomère B. On peut ainsi estimer le pourcentage d'isomère B dans le mélange par la formule suivante:

$$\%B = \frac{h_2}{h_1 + h_2}$$

dans laquelle $h_1$ représente la hauteur du pic $P_1$ et $h_2$ représente la hauteur du pic $P_2$.

Après cette évaluation, on ajoute au mélange d'isomères une quantité de solvant appropriée et la quantité voulue de nitrate d'uranyle qui correspond à environ 3,5 mol de nitrate d'uranyle par mole d'isomère B dans le cas du DCH18C6, pour précipiter le complexe nitrate d'uranyle-isomère B.

La quantité de solvant utilisée dépend de la nature du solvant. Dans le cas de l'heptane, on peut utiliser environ 40 ml de solvant par gramme de mélange d'isomères.

On peut réaliser la réaction de précipitation à la température ambiante, de préférence sous agitation, et l'on opère pendant une durée suffisante, par exemple 24h, pour précipiter la totalité des complexes nitrate d'uranyle - autres isomères que l'isomère A.

Lorsque la précipitation est achevée, on filtre la solution pour recueillir, d'une part, une solution de l'isomère A pur dans le solvant et, d'autre part, un précipité comprenant le complexe nitrate d'uranyle-isomère B.

Pour obtenir l'isomère A pur, on soumet la solution à une évaporation.

Pour récupérer l'isomère B, on décompose le complexe par traitement à l'eau et au chloroforme, ce qui permet de récupérer également le nitrate d'uranyle.

Les exemples qui suivent illustrent la séparation des isomères du DCH18C6 (exemples 1 à 9) et du DCH24 et l'utilisation de l'isomère cis-syn-cis du DCH18C6 pour extraire le plutonium (exemple 11).

**Exemple 1.**

On dissout 24,6 g de DCH18C6 contenant 62,9% d'isomère A (15,47 g), 37% d'isomère B (9,1 g) et moins de 1% des autres isomères dans 74 ml d'heptane et on recristallise l'isomère B à partir de cette solution pendant 24 h à la température ambiante. On recueille ainsi par filtration 3,4 g d'isomère B cristallisé.

On soumet ensuite la solution à une évaporation et l'on recueille 21,2 g d'un mélange d'isomères ayant maintenant une teneur en isomère A d'environ 73%.

On ajoute alors à ce mélange 850 ml d'heptane et 108 ml d'une solution de nitrate d'uranyle $(NO_3)_2UO_2$, $6H_2O$ à 25% en poids et on soumet l'ensemble à une agitation, pendant 24h, à la température ambiante. On filtre ensuite le précipité formé, puis on le sèche à l'étuve à 60 °C pendant 20 h. On obtient ainsi 12 g de précipité que l'on dissout dans 300 ml de chloroforme et 150 ml d'eau distillée. On sèche ensuite la phase organique sur $MgSO_4$, on filtre et on évapore le solvant sous vide, ce qui donne 5,6 g d'isomère B, soit un rendement de 98,9% et on récupère l'isomère A à partir de la solution de filtration par évaporation du solvant. On obtient ainsi 15,4 g d'isomère A, soit un rendement de 99,5%.

Ce procédé permet donc de recueillir les isomères A et B de façon quantitative avec un rendement supérieur à 99% pour l'isomère A.

Par ailleurs, on remarque que cette méthode n'utilise que des produits peu coûteux et que l'on peut récupérer également quantitativement le nitrate d'uranyle qui a été utilisé.

**Exemples 2 à 8.**

Dans ces exemples, on suit le même mode opératoire que dans l'exemple 1 pour réaliser la précipitation d'un complexe nitrate d'uranyle - isomère B à partir de 500 mg d'un mélange d'isomères du DCH18C6 contenant 68,8% d'isomère A (344 mg) en utilisant 20 ml d'heptane, des quantités différentes de solution de nitrate d'uranyle afin d'avoir des rapports molaires nitrate d'uranyle / isomère B différents et une durée de précipitation de 3 h dans les exemples 2 à 7 et de 24h dans l'exemple 8.

Dans chaque cas, on détermine le pourcentage d'isomère A obtenu dans la solution séparée après évaporation du solvant.

Les résultats et les conditions de ces exemples sont donnés dans le tableau annexé.

Au vu des résultats de ce tableau, on constate que seul l'exemple 8 conduit à une pureté de 100% en isomère A de la solution recueillie après filtration du précipité. Ceci correspond à un rapport molaire nitrate d'uranyle / isomère B de 3,6 et à une durée d'agitation de 24h.

Ainsi, le procédé de l'invention est très avantageux puisqu'il permet de séparer quantitativement les isomères du DCH18C6 et de récupérer l'isomère A avec un degré de pureté de 100%.

## Exemple 9.

Dans cet exemple, on dissout du DCH18C6 contenant 54% d'isomère A et 46% d'isomère B dans 3 volumes d'heptane et on recristallise l'isomère B à partir de cette solution à -5°C. On recueille ainsi par filtration une quantité de l'isomère B représentant 11,8% du mélange de départ.

On soumet la solution à une évaporation et l'on recueille un mélange d'isomères ayant une teneur en isomère A de 66% et une teneur en isomère B de 34%.

On ajoute au mélange 8 volumes d'heptane et 2 volumes de $UO_2^{2+}$ en 2 heures puis on soumet à une agitation pendant 48 h.

On recueille le précipité par filtration et on le dissout dans de l'eau et du chloroforme. On soumet la solution organique obtenue à une distillation, ce qui donne l'isomère B avec un titre en B de 97%.

On récupère l'isomère A à partir de la solution de filtration par évaporation avec un titre en A de 90%.

## Exemple 10.

Dans cet exemple, on part de 1 g d'un mélange d'isomères du DCH24C8, soit de l'éther-couronne de formule (I) avec n = 2 contenant 47% d'isomère cis-anti-cis et 53% d'isomère cis-syn-cis, que l'on dissout dans 3 ml d'heptane et on y ajoute 1,5 ml de nitrate d'uranyle $UO_2(NO_3)_2,6H_2O$ sous agitation.

On sépare par filtration le précipité formé et on le dissout dans un mélange d'eau et de chloroforme. On sèche ensuite la phase organique sur $MgSO_4$ puis on évapore le solvant sous vide; on récupère ainsi l'isomère cis-anti-cis.

On récupère ensuite l'isomère cis-syn-cis dans la solution d'heptane séparée.

L'isomère A du DCH18C6 peut être utilisé avec intérêt à la place du mélange d'isomères (A et B), notamment pour l'extraction du plutonium dans les procédés de retraitement de combustibles nucléaires irradiés comme le montre l'exemple donné ci-après.

## Exemple 11.

Dans cet exemple, on traite une solution aqueuse nitrique contenant:

- 906mg/l d'uranium (VI)
- 1360mg/l de plutonium (IV)
- $18,1.10^7$ Bq/l (4,88mCi/l) de produits de fission, et
- 5mol/l de $HNO_3$

par un solvant organique constitué par du chloroforme contenant 0,134 mol/l d'isomère cis-syn-cis du DCH18C6 obtenu dans l'exemple 1.

Dans ce but, on met en contact 15 ml de la solution aqueuse avec 30ml du solvant organique d'extraction, sous agitation, pendant 10min. On sépare ensuite les deux phases par décantation, on mesure leurs teneurs respectives en uranium, et en plutonium et en produits de fission, et on calcule les coefficients de partage Dm de l'uranium, du plutonium et des produits de fission entre les deux phases. Ce coefficient de partage Dm correspond au rapport de la concentration de l'élément dans le solvant organique sur la concentration du même élément dans la solution aqueuse.

Les résultats obtenus sont donnés dans le tableau 2 qui suit.

Dans ce tableau, on a également indiqué les valeurs des constantes d'extraction Kex du plutonium et de l'uranium qui ont été calculées à partir des valeurs obtenues.

Au vu de ce tableau, on remarque que les coefficients de partage et d'extraction du plutonium sont beaucoup plus importants que ceux de l'uranium.

A titre comparatif, on a donné également dans le tableau 2 les résultats obtenus en traitant la même solution aqueuse avec deux solvants organiques constitués respectivement par du chloroforme contenant 0,134mol/l d'isomère cis-anti-cis du DCH18C6 et par du chloroforme contenant 0,134mol/l du mélange d'isomères du DCH18C6 du commerce.

Au vu de ce tableau 2, on constate que l'isomère cis-syn-cis permet d'atteindre des résultats bien supérieurs à ceux obtenus avec l'isomère cis-anti-cis et le mélange d'isomères.

TABLEAU 1

| Essai | heptane (mL) | DCH18C6 à 68,8% A (mg) | solution de $(NO_3)_2UO_2$, $6H_2O$ à 25% (mL) | mole de $UO_2^{2+}$ mole de B | durée de l'agitation (h) | % en A dans le produit solubilisé |
|---|---|---|---|---|---|---|
| 2 | 20 | 500 | 1 | 1,2 | 3 | 80,3 |
| 3 | 20 | 500 | 2 | 2,4 | 3 | 87,4 |
| 4 | 20 | 500 | 3 | 3,6 | 3 | 91,9 |
| 5 | 20 | 500 | 4 | 4,7 | 3 | 92,9 |
| 6 | 20 | 500 | 5 | 5,9 | 3 | 93,2 |
| 7 | 20 | 500 | 7 | 8,3 | 3 | 95,1 |
| 8 | 20 | 500 | 3 | 3,6 | 24 | 100 |

**TABLEAU 2**

| | Dm (Pu) | $D_m$ (U) | Dm (PF) | Kex Pu $mol^{-6} l^{6}$ | Kex U $mol^{-3} l^{3}$ |
|---|---|---|---|---|---|
| 0,134 mol/l cis-syn-cis dans $CHCl_3$ | 51 | 1,9 | 0,22 | 477 | 6,4 |
| 0,134 mol/l de cis-anti-cis dans $CHCl_3$ | 20 | 1,5 | 0,012 | 140 | 4,0 |
| 0,134 mol/l DCH 18C6 du commerce dans $CHCl_3$ | 23 | 1,6 | 0,014 | 250 | 4,0 |

## Revendications

1. Procédé de séparation des isomères d'un éther-couronne de formule:

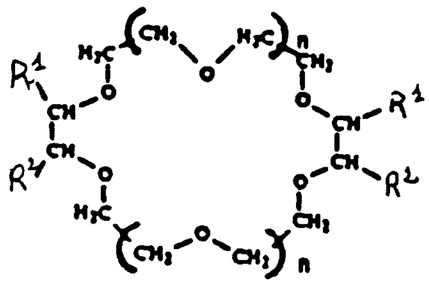

(I)

dans laquelle n = 0 ou est un nombre entier allant de 1 à 3, $R^1$ et $R^2$ qui sont identiques ou différents, représentent un radical alkyle, alcoxyalkyle ou poly(alcoxyalkyle), ou $R^1$ et $R^2$ forment ensemble un radical cycloalkyle,
caractérisé en ce qu'il comprend les étapes successives suivantes :
a) dissoudre dans un solvant organique un mélange des isomères dudit éther-couronne comprenant l'isomère cis-anti-cis et l'isomère cis-syn-cis,
b) ajouter du nitrate d'uranyle à la solution obtenue dans l'étape a) en quantité telle que l'on précipite pratiquement tous les isomères de l'éther-couronne sauf l'isomère cis-syn-cis, sous la forme de complexes avec le nitrate d'uranyle,
c) séparer le précipité ainsi formé, et
d) récupérer l'isomère cis-syn-cis pur restant dans la solution.

2. Procédé selon la revendication 1, caractérisé en ce que l'on récupère l'isomère cis-anti-cis à partir du précipité séparé dans l'étape c).

3. Procédé selon l'une quelconque des revendications 1 et 2, caractérisé en ce qu'il comprend une étape préalable consistant à séparer une partie de l'isomère cis-anti-cis présent dans le mélange d'isomères de départ, par dissolution du mélange dans un solvant organique, cristallisation de l'isomère cis-anti-cis présent dans cette solution et séparation de l'isomère cis-anti-cis ainsi cristallisé.

4. Procédé selon l'une quelconque des revendications 1 et 3, caractérisé en ce que le solvant organique est un hydrocarbure saturé aliphatique ou un hydrocarbure aromatique.

5. Procédé selon la revendication 4, caractérisé en ce que le solvant organique est l'heptane.

6. Procédé selon l'une quelconque des revendications 1 à 5, caractérisé en ce que l'éther-couronne répond à la formule:

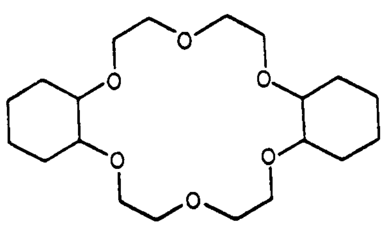

(II)

7. Procédé selon la revendication 6, caractérisé en ce que la quantité de nitrate d'uranyle correspond à 3,5 mol de nitrate d'uranyle $(NO_3)_2UO_2, 6H_2O$ par mol d'isomère cis-anti-cis.

## Claims

**1.** Method for separating isomers of a crown ether with the formula:

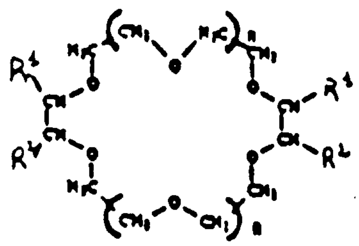

(I)

in which n = 0 or is a whole number ranging from 1 to 3, $R_1$ and $R_2$, which are identical or different, represent an alkyl, alcoxyalkyl or poly(alcoxyalkyl) radical or $R_1$ and $R_2$ collectively form a cycloalkyl radical, characterized in that it comprises the following successive stages:

a) dissolving in an organic solvent a mixture of the isomers of said crown ether iincluding the cis-anti-cis isomer and the cis-syn-cis isomer,

b) adding uranyl nitrate to the solution obtained in stage a) in sufficient quantities so that virtually all the isomers, except the cis-syn-cis isomer, of the crown ether are precipitated in the form of complexes along with the uranyl nitrate,

c) separating the thus formed precipitate, and

d) recovering the pure cis-syn-cis isomer remaining in the solution.

**2.** Method according to claim 1, characterized in that the cis-anti-cis isomer is recovered from the precipitate separated in stage c).

**3.** Method according to claim 1 or 2, characterized in that it comprises a preliminary stage consisting of separating one portion of the cis-anti-cis isomer present in the initial isomer mixture by dissolving the mixture in an organic solvent, crystallizing the cis-anti-cis isomer present in this solution and separating the thus crystallized cis-anti-cis isomer.

**4.** Method according to any one of claims 1 to 3, characterized in that the organic solvent is a saturated aliphatic hydrocarbon or an aromatic hydrocarbon.

**5.** Method according to claim 4, characterized in that the organic solvent is heptane.

**6.** Method according to any one of claims 1 to 5, characterized in that the crown ether responds to the formula:

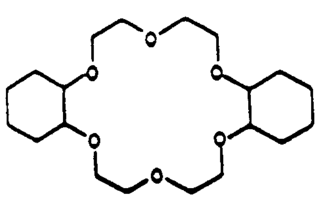

(II)

**7.** Method according to claim 6, characterized in that the uranyl nitrate quantity corresponds to 3.5 moles of uranyl nitrate $(NO_3)_2UO_2,6H_20$ per mole of the cis-anti-cis isomer.

## Patentansprüche

**1.** Verfahren zur Abtrennung von Isomeren von einem Ätherring mit der Formel:

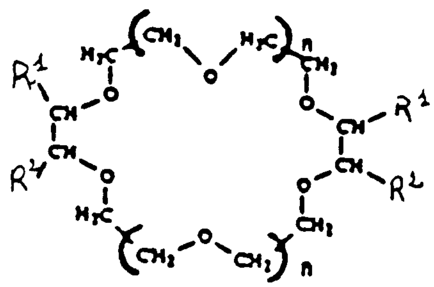

(I)

bei dem n Null oder einem ganzzahligen Wert zwischen 1 und 3 entspricht, wobei $R^1$ und $R^2$ identisch oder verschieden sein können und ein Alkyl-, Alkoxyalkyl- oder (Poly)alkoxyalkylradikal bilden, oder bei dem $R^1$ und $R^2$ zusammen ein Zykloalkylradikal bilden und
das dadurch gekennzeichnet ist, daß es die im folgenden genannten Arbeitsschritte umfaßt:

a) Auflösung eines Gemisches aus Isomeren mit der o.g. Ätherringstruktur, das aus Isomeren vom Typ cis-anti-cis und cis-syn-cis besteht, in einem organischen Lösungsmittel,

b) Beimengung von Uranylnitrat zu der in Arbeitsschritt a) erhaltenen Lösung in der Menge, in der man gewöhnlich alle Isomere mit Ätherringstruktur mit Ausnahme der cis-syn-cis-Isomere in Form von Komplexen mit Uranylnitrat ausfällt,

c) Abscheiden des auf diese Weise gebildeten Niederschlages
und

d) Rückgewinnung des in der Lösung verbleibenden, reinen cis-syn-cis-Isomers.

**2.** Verfahren nach Anspruch 1, dadurch gekennzeichnet, daß das cis-anti-cis-Isomer aus dem in Arbeitsschritt c) abgeschiedenen Niederschlag gewonnen wird.

**3.** Verfahren nach einem der Ansprüche 1 und 2, dadurch gekennzeichnet, daß es einen Vorarbeitsgang umfaßt, indem durch Auflösung des Gemisches in einem organischen Lösungsmittel, durch die Auskristallisierung des in dieser Lösung enthaltenen cis-anti-cis-Isomers und die Abscheidung des auf diese Weise auskristallisierten cis-anti-cis-Isomers ein Teil des in dem Isomerausgangsgemisches enthaltenen cis-anti-cis-Isomers abgeschieden wird.

**4.** Verfahren nach einem der Ansprüche 1 und 3, dadurch gekennzeichnet, daß als organisches Lösungsmittel eine gesättigte azylische Kohlenwasserstoffverbindung oder eine aromatische Kohlenwasserstoffverbindung verwendet wird.

**5.** Verfahren nach Anspruch 4, dadurch gekennzeichnet, daß als organisches Lösungsmittel Heptan verwendet wird.

**6.** Verfahren nach einem der Ansprüche 1 bis 5, dadurch gekennzeichnet, daß der Ätherring der folgenden Formel entspricht:

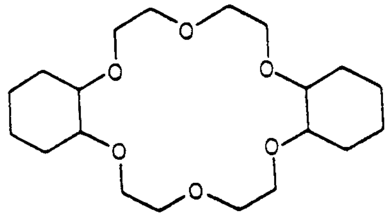

(II)

**7.** Verfahren nach Anspruch 6, dadurch gekennzeichnet, daß die Uranylnitratmenge einem Wert von 3,5 mol Uranylnitrat $(NO_3)_2UO_2, {}_6H_2O$ pro einem Mol cis-anti-cis-Isomer entspricht.

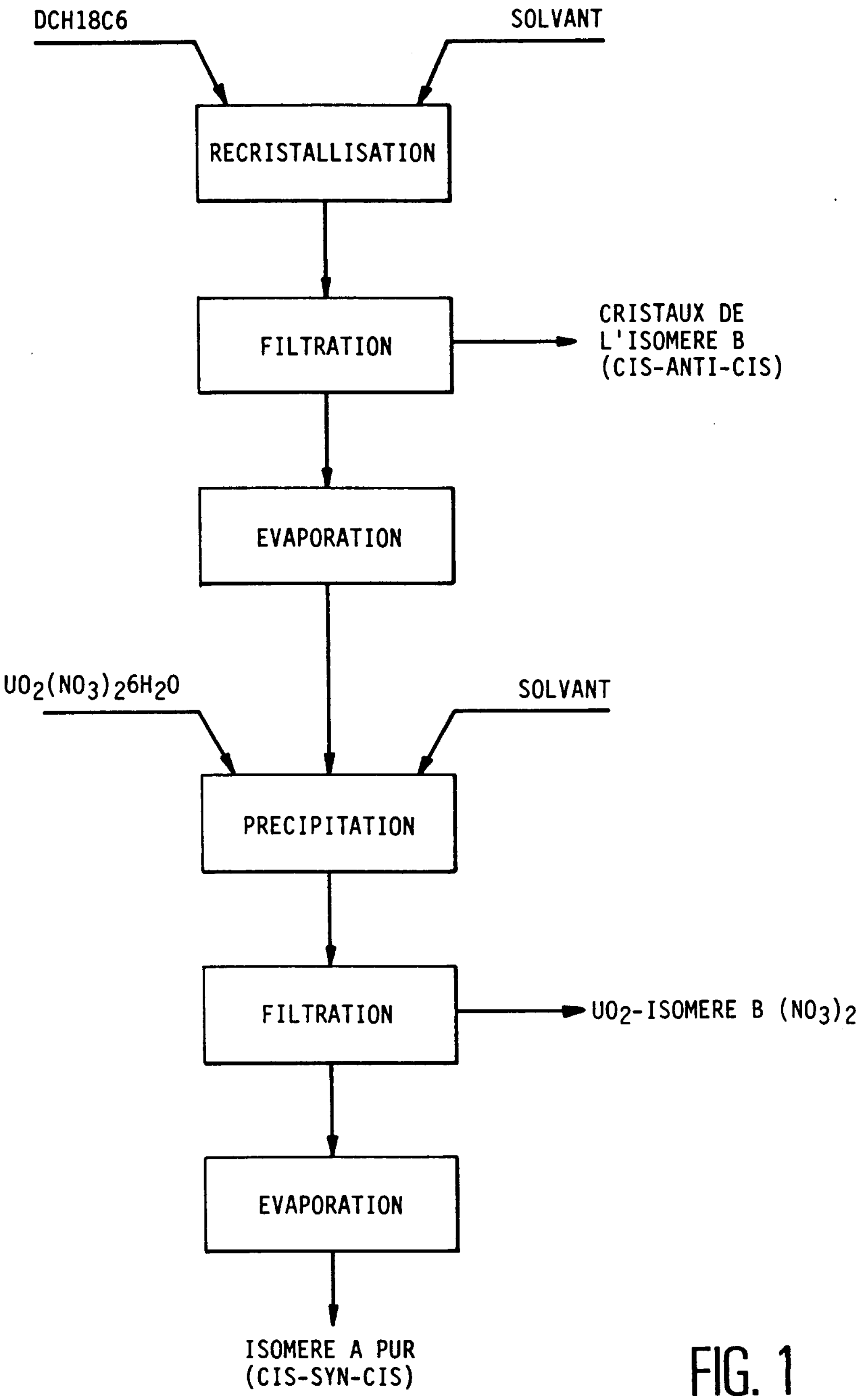
DCH18C6
SOLVANT
RECRISTALLISATION
FILTRATION
CRISTAUX DE L'ISOMERE B (CIS-ANTI-CIS)
EVAPORATION
$UO_2(NO_3)_2 6H_2O$
SOLVANT
PRECIPITATION
FILTRATION
$UO_2$-ISOMERE B $(NO_3)_2$
EVAPORATION
ISOMERE A PUR (CIS-SYN-CIS)

FIG. 1

FIG. 2